# EUROPEAN PATENT SPECIFICATION

(11) **EP 4 301 293 B1**
(45) Date of publication and mention of the grant of the patent: **06.05.2026**
(21) Application number: 22764207.1
(22) Date of filing: 05.03.2022
(51) Int. Cl.: A61F 5/02, A61F 5/30, B33Y 80/00

(54) **ADJUSTABLE MULTI-BAND SPINE BRACE SYSTEM WITH AN OPTIONAL SLIDABLE INTERNAL LATERAL PANEL**
EINSTELLBARES MEHRBAND-RÜCKENSTÜTZSYSTEM MIT OPTIONAL VERSCHIEBBARER INNERER SEITENPLATTE
ORTHÈSE DORSALE MULTIBANDE RÉGLABLE À PANNEAU LATÉRAL INTERNE COULISSANT FACULTATIF

(30) Priority: 05.03.2021 US 202163157424 P; 05.03.2021 US 202163157441 P; 04.03.2022 US 202217687567; 04.03.2022 US 202217687592
(43) Date of publication of application: 10.01.2024
(73) Proprietor: Aspen Medical Products, LLC, Irvine, CA 92618 (US)
(72) Inventor: ROMO, Albert V., Irvine, CA 92618 (US); PRICE, Jane, Irvine, CA 92618 (US); WONG, Geoffrey, Irvine, CA 92618 (US)
(74) Representative: Dehns
(86) International application number: PCT/US2022/019049
(87) International publication number: WO 2022/187722

(56) References cited:
- WO-A1-03/088877
- WO-A1-2005/117769
- GB-A- 2 525 004
- US-A- 5 690 609
- US-A1- 2011 105 971
- US-A1- 2013 237 891
- US-A1- 2014 058 307
- US-A1- 2014 228 727
- US-A1- 2019 343 673
- US-B1- 6 336 908
- US-B1- 6 419 652
- US-B2- 7 473 235

## Description

### CROSS-REFERENCE TO RELATED APPLICATIONS

This application claims priority to U.S. Application No. 17/687567 filed March 4, 2022, which claims the benefit of priority on U.S. Provisional Patent Application No. 63/157,424 filed March 5, 2021, and U.S. Application No. 17/687,592 filed March 4, 2022 which claims the benefit of priority on U.S. Provisional Patent Application No. 63/157441 filed March 5, 2021.

### FIELD

Embodiments of the disclosure relate to the field of medical devices. More specifically, one embodiment of the disclosure relates to an adjustable spine braces and components thereof.

### GENERAL BACKGROUND

The following description includes information that may be useful in understanding the described invention. It is not an admission that any of the information provided herein is prior art or relevant to the presently claimed invention, or that any publication specifically or implicitly referenced is prior art.

Orthopedic braces (orthoses) usually need to be adjusted or customized in some manner to conform to the body part(s) being braced, and then properly positioned. A typical orthosis commonly has at least two portions, a rigid portion supporting a body part, and a flexible or semi-flexible portion securing the orthosis to the body. Various types of spine braces, such as a thoracic-lumbar-sacral orthosis (TLSO) for example, are used to provide support and stabilization of the spine normally after a back injury and/or surgery, and in some cases, may be utilized to address spinal pathologies. A TLSO is a brace that limits movement in a wearer's spine from the thoracic area (mid-back area) to the wearer's sacrum (lower-back area).

Conventional adjustable braces typically have a narrow anterior region, i.e., the front, and the area where the adjustment is done is typically on the side. A loop-and-hook mechanism is usually used to shorten/adjusting the length of the brace. The adjustment process is vital, as a proper fit of the brace provides the wearer with improved pain reduction and promotes healing. However, one common problem with the adjustment process is that the brace needs to apply the proper compression and stabilization to achieve pain reduction and promoted healing. If the circumference is inadequate or the contours of the belt cannot accommodate the user, the fit (e.g., proper compression and stabilization) will be inadequate. Therefore, there is a need for a spine brace that is easily adjustable to provide proper fit to the wearer. WO 03/088877 relates to a low-profile lumbo-sacral orthosis.

### BRIEF DESCRIPTION OF THE DRAWINGS

The invention is defined in claim 1.

Embodiments of the invention are illustrated by way of example and not by way of limitation in the figures of the accompanying drawings, in which like references indicate similar elements and in which:
**FIG. 1A** is a perspective outside view of an exemplary embodiment of a spine brace system.
**FIG. 1B** is a perspective inside view of an exemplary embodiment of a spine brace system of **FIG. 1A****.**
**FIG. 2** is an exemplary embodiment of three bands of the spine brace system of **FIG. 1A** and **FIG. 1B****.**
**FIG. 3A** is a top view of an exemplary embodiment of an adjustment mechanism featuring a pulley system and slots implemented with the spine brace system of **FIG. 1A** and **FIG. 1B****.**
**FIG. 3B** is a top view of an angled configuration of the upper and lower slots with respect to the middle slot implemented within the adjustment mechanism of the spine brace system of **FIG. 1A** and **FIG. 1B****.**
**FIG. 4A** is a top view of an exemplary embodiment of the first side pulley system implemented with the spine brace system of **FIG. 1A** and **FIG. 1B****.**
**FIG. 4B** is a top view of an exemplary embodiment of the second side pulley system implemented with the spine brace system of **FIG. 1A** and **FIG. 1B****.**
**FIG. 5** is a perspective outside view of an exemplary embodiment of a spine brace system.
**FIG. 6** is a perspective outside view of a first exemplary embodiment of a spine brace system with slidable, internal lateral panel.
**FIG. 7** is a perspective outside view of a second exemplary embodiment of the spine brace system with slidable, internal lateral panel of **FIG. 6****.**
**FIG. 8** is a perspective outside view of a third exemplary embodiment of the spine brace system with slidable, internal lateral panel of **FIG. 6****.**

### DETAILED DESCRIPTION

As noted above, conventional adjustable spine braces are adjusted on the side via a loop-and-hook mechanism. Further, conventional braces provide for partial or substantial immobilization of the torso, back or other portion of a wearer's body. The thoracic lumbar sacral orthosis back brace with adjustable sliding back panel fail to use any lateral panels. There is a need for an improved brace with repositionable lateral panels that provide support, comfort and ease of use to the patient. In order to tackle this issue, a brace with slidable, internal lateral panel is disclosed. Herein, the circumference and contour of the brace can be adjusted to proper relevant circumferential lengths.

Disclosed herein is a spine brace featuring a first arm covering a first side of a patient wearing the spine brace and a second arm covering a second side of the patient, respectively. Each arm features a middle band disposed between an upper band and a lower band, and an adjustment mechanism that allows for independent adjustment of each band. Each arm may feature a cord coupled to the middle band to tighten the spine brace. The adjustment mechanism may include slots for each of the bands, where an upper slot for the upper band and a lower slot for the lower band are angled with respect to a middle slot that is shaped with a narrowing toward a center region of the middle slot. The first and second arms are removably coupled together using a hook and loop coupling or any other suitable coupling mechanism.

As described herein, the adjustment mechanism may be deployed as one or more components of a tightening mechanism such as components of a pulley base forming a part of a pulley system. According to one embodiment of the disclosure, the adjustment mechanism features upper, middle and lower slots formed into a body of the pulley base. The upper and lower slots may be angled with respect to the middle slot to receive the upper band and lower band so that the spine brace may be tapered from a first side closer to the posterior portion of the spine brace to a second side closed to the anterior portion of the spine brace. As described herein, the "spine brace" may constitute any type of lumbar sacral orthosis (LSO), including a standard LSO (lower back brace belt) or other orthoses configured to provide greater patient immobilization, such as a LSO, TLSO or CTLSO as described below.

When deployed as a LSO, TLSO or CTLSO, the brace may be further configured in accordance with the tightening mechanism that includes slots for each of the upper, middle and lower bands.

Each of the components described herein may be formed, at least in part, with a rigid material, such as hardened plastic for example, to provide greater stiffness for immobilization of the wearer, e.g., the patient. In various embodiments, each band is made of different materials. For example, the upper and lower bands are made of a material which may be softer (e.g., provide smoother or cushiony texture based on greater flexibility, looser knit or knit pattern, different chemical composition, different material structure, or the like) than the material from which the middle band is made. Alternatively, at least two of the bands are made of the same type of material. Herein, the posterior portion of the spine brace is shaped and sized to accommodate the mid-to-lower portions of the back to partially immobilize the patient's spine. When worn, the posterior portion is oriented to reside or is substantially in parallel with a frontal plane located at the posterior surface of the patient. It provides support along the thoracic, lumbar and sacral regions of the patient's spine. A lateral portion of each belt arm of the spine brace may cover one side of the patient. Additionally, the anterior portion resides or substantially is parallel with a frontal plane on the anterior surface of the patient, and is in contact with the abdominal region and anterior rib cage of the patient.

The anterior portion of each of the first and second arms may include a coupling section to removably attach to the other anterior portion, e.g., via hook fasteners such as VELCRO^{®} strips. Alternatively, the anterior portion of each of the first and second arms may be removably coupled together by using a hook and loop coupling. The spine brace may be slightly tapered laterally from one side to the other side, in that, while the middle band's width is substantially constant, the upper/lower bands are extended slightly downward/upward to couple the middle band. In some embodiments, this tapered configuration improves the contour of the spine brace and enhances the comfort of the patient.

The tightening mechanism may include a plurality of slots for the bands. The upper and lower slots may be angled with respect to the middle slot to allow the upper band and lower band to extend slightly towards each other to couple with the middle band and forming a slightly tapered spine brace.

In some embodiments, a separate tightening mechanism can be used for each of the first and second arms of the spine brace. Each tightening mechanism may be designated to allow independent tightening of the top or bottom of the corresponding arm.

To facilitate convergence of the upper and lower bands and forming a slightly tapered spine brace, a lower part of the upper slot slightly overlaps with an upper part of the middle slot, and an upper part of the lower slot slightly overlaps with a lower part of the middle slot. The middle slot features an opening with a selected shape, generally referred to as a "T-shaped" opening merely for clarity, which narrows slightly in the center of the tightening mechanism which can further guide the middle cord.

According to some embodiments, the spine brace system may feature one or more pulley bases of a pulley system, each operating as the tightening mechanism. The pulley system featuring a cord and a pair of pulley bases, where each pulley base includes a plurality of pulleys. By tightening the cord that passes through each of the pulleys, the patient can tighten a corresponding upper or lower circumference of the spine brace system.

Some embodiments of the present disclosure generally relate to a lumbar brace including slidable, internal lateral panels. According to an embodiment of the disclosure, the lumbar brace is configured to wrap around a lumbar region of a wearer and features a first arm around a first side of the wearer, and a second arm around a second side of the wearer. Each arm features a first band, a panel slidably disposed at a lateral side of the wearer and coupled to the first band, and a loop connector coupled to the panel. The first band has a horizontally extending channel, within which the panel moves. Configured to provide lateral support for the wearer, the panel features a rigid material, and the loop connector is tethered to the panel.

Herein, rotational movement of the connector loop causes lateral movement of the panel, which adjusts a location of the panel around the lumbar region of the wearer. The rotational movement of the connector loop may be restricted to restrain lateral movement of the panel to a prescribed area of the first band. According to various embodiments of the present disclosure, the location of the panel is adjusted based on the size of the wearer of the brace. In general, while a first position might be desirable, e.g., comfortable, for a first wearer, the same position is undesirable, e.g., uncomfortable, for another wearer who is larger in size compared to the first wearer.

In various embodiments of the present disclosure, one or more tubes, wires, cables, cords or other interconnects may be inserted into the channel. For example, wires or cords connected to medical equipment and monitors and/or tubes, e.g., catheters, can be inserted into the channel for wire/tube management.

In embodiments, moving the handle member may cause the panel to move horizontally within the channel, thus the wearer can adjust the location of the panel. In particular, based on a size of the wearer, the desired location of the panel changes. The handle member helps the wearer to easily adjust the panel and reposition the panel to a desired location.

According to another embodiment of the disclosure, the lumbar brace is configured to wrap around a lumbar region of a wearer and features a first band, and a panel slidably disposed within a channel on the first band. A lateral movement of the panel in the channel is constrained by a first and a second boundary at a first and a second end of the channel, respectively. The lumbar brace may feature a handle member disposed on the panel. The handle member may be a tube knitted to the panel, a leash or any other suitable handle. The lumbar brace may further feature an upper and a lower band, which the first band may be disposed between an upper band and a lower band. The panel includes a rigid material, and the lateral movement of the panel adjusts a location of the panel around the lumbar region of the wearer.

According to yet another embodiment of the disclosure, the lumbar brace is configured to wrap around a lumbar region of a wearer and features a first band having has a horizontally extending channel, a panel slidably disposed at a lateral side of the wearer within the channel, and a plurality of openings disposed on the first band. The panel is laterally slidable between the plurality of openings. The lumbar brace may further feature a second band and a third band disposed on upper side and lower side of the first band, respectively. The channel may extend along the entire length of the first band, and the plurality of openings may be disposed along the channel. Sliding the panel between the plurality of openings may adjust the location of the panel around the lumbar region of the wearer.

Each of the components described herein may be formed, at least in part, with a rigid material, such as hardened plastic for example, to provide greater stiffness for immobilization of the patient. Herein, the posterior portion of the lumbar brace is shaped and sized to rest against the mid-to-lower portions of the patient's back to partially immobilize the spine of the patient. When worn, the posterior portion is oriented to reside or is substantially in parallel with a back plane of the patient and provide support along a thoracic and lumbar regions of the patient's spine. A lateral portion of each arms of the lumbar brace may cover one side of the patient. An anterior portion resides or substantially is parallel with a frontal plane of the patient, and is contact with the abdominal region of the patient's spine. It is contemplated that each of the first and second arm includes a posterior portion covering the back of the wearer, a lateral portion covering the sides of the wearer, and an anterior portion covering the front of the wearer.

Each arm's anterior portion may include a coupling section to removably attach to the other anterior portion, e.g., via hook fasteners such as VELCRO^{®} strips. Alternatively, the arms' anterior portions are removably coupled together by using a hook and loop coupling.

### I. TERMINOLOGY

In the following description, certain terminology is used to describe aspects of the invention. For example, the term "member" may be construed as a structural component of an orthopedic brace. In certain situations, a member may include a component covered by soft goods such as one or more textiles, one or more fabrics (woven fabrics and/or non-woven fabrics), leathers, and/or another covering material. These soft goods may feature "loop" type fasteners or other variants to which a "hook" type fastener may be attached or may feature a hook-type fastener for attachment to a loop-type fastener. In other situations, the member may be soft goods attached to another structural component of the orthopedic brace such as a textile or fabric sewn to form together such as a knit textile with pockets in which the structural component(s) can be positioned within the pocket(s).

The term "attach" and other tenses of the term (attached, attaching, etc.) may be construed as a physical connection activity, such as physically connecting a first member to a second member. A "fastener" may be construed as any physical component that is used to attach different members together. An illustrative example of different types of fasteners and fastening techniques may include, but are not limited or restricted to snaps, buttons, clasps, buckles, adhesives, sewing, heat sealing (or melting), gluing, knitting, or other physical coupling techniques such as a hook and loop connection.

The terms "rigid" or "rigidity" with respect to a member or portion of a member may be construed as the member being configured to at least partially resist bending or deformation. According to this definition, different lengths of a given structure and composition can be rigid at a shorter length, and flexible at a longer length. As used herein, the term "rigid" with respect to a member or portion of a member may be construed as the member will be permanently deformed or broken if bent or twisted by at least 90°.

The term "softer" may pertain to a characteristic of a first material when compared to a second material, where the level of softness may be a result of differences in the first material from the second material to provide the first material with a smoother or cushiony nature, achievable by the first material having greater flexibility caused by a looser knit, a higher thread count, a different chemical composition, and/or a different structural composition.

The term "channel" is a partially-enclosed housing, namely a structure having partially enclosed perimeter except for at least one opening at an end of the channel (and perhaps two openings at the end of the channel). As a result, a partially-enclosed chamber operates as a structure that is configured to secure, maintain and protect components, such as a lateral panel or one or more tubes, wires, cables, cords or other interconnects passing there through.

Finally, the terms "or" and "and/or" as used herein are to be interpreted as inclusive or meaning any one or any combination. As an example, "A, B or C" or "A, B and/or C" mean "any of the following: A; B; C; A and B; A and C; B and C; A, B and C." An exception to this definition will occur only when a combination of elements, functions, steps or acts are in some way inherently mutually exclusive.

As this invention is susceptible to embodiments of many different forms, it is intended that the present disclosure is to be considered as an example of the principles of the invention and not intended to limit the invention to the specific embodiments shown and described.

### II. GENERAL ARCHITECTURE - MULTI-BAND SPINE BRACE SYSTEM

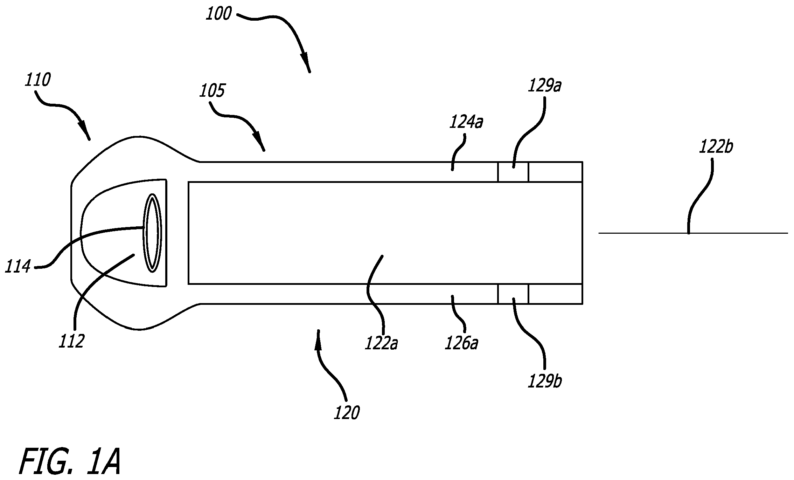

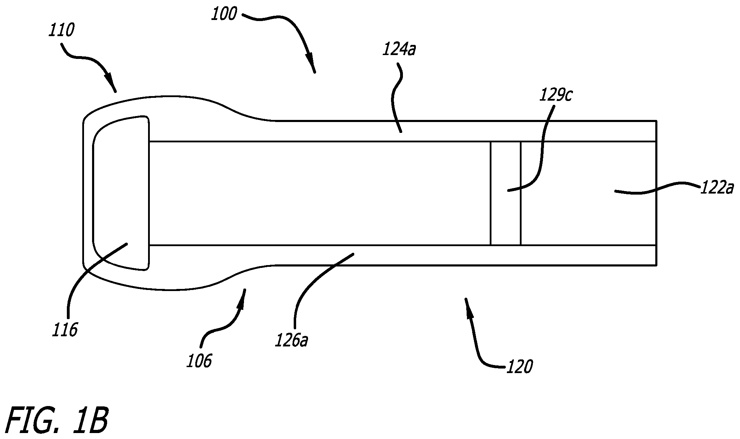

Referring to **FIG. 1A****,** a perspective outside view of an exemplary embodiment of an adjustable multi-band spine brace system 100 is shown. For sake of simplicity, a first arm 105 of the spine brace system 100 is shown. Each arm of the spine brace system 100, such as the first arm 105 of **FIG. 1A** and a second arm 106 of **FIG. 1B****,** includes an anterior portion 110 (i.e., a front portion), a lateral portion 120, and a posterior portion (i.e., a back portion, not shown). The posterior portion is oriented, when worn, to generally reside or is substantially in parallel with a back plane of the patient. The posterior portion may provide support along a thoracic and lumbar regions of the patient's spine. The lateral portion 120 may cover the sides of the patient. Similarly, the anterior portion 110 is oriented, when worn, to generally reside or is substantially in parallel with a frontal plane of the patient. The anterior portion 110 may be in contact with the abdominal region and rib cage of the patient's spine.

Each arm may include a coupling section, such as a coupling 112 for the first arm 105 shown in **FIG. 1A****,** and a coupling 116 for the second arm 106 shown in **FIG. 1B****,** respectively. Each of the coupling sections 112 and 116 is configured to removably attach/couple to the other coupling section. Regardless of the size and shape, the coupling sections 112/116 of the arms 105/106 are so formed that match each other and, when attached/coupled together, form a uniformly closed brace which circumferentially surrounds the body of the patient. Therefore, in some embodiments, the anterior portions 110 of the two arms 105 and 106 of the spine brace system 100 are removably coupled together by using a hook and loop coupling. As a non-limiting example, the coupling section 112 may include a VELCRO^{®} strip or another type of hook fastener while the coupling section 116 may include a loop fastener. In some embodiments, the anterior section 110 may further include a slot 114. This creates a pocket to facilitate better purchase of the fingers during donning.

As shown, the spine brace system 100 further features a middle band 122a disposed between an upper band 124a and a lower band 126a. According to one embodiment of the disclosure, the middle band 122a, the upper band 124a and the lower band 126a are attached/coupled only in one end (e.g., at the posterior portion). That is, the middle band 122a, the upper band 124a and the lower band 126a can operate independent of each other. Each of the upper band 124a, the lower band 126a and the middle band 122a are made of flexible materials that can flex and/or bend around the circumference body of the patient.

In various embodiments, each band 122a, 124a and 126a may be made of different materials. For example, the upper and lower bands 124a and 126a may be made of a same material which may be substantially softer than the material from which the middle band 122a is made. Alternatively, at least two of the bands 122a/124a, 122a/126a, 124a/126a, 122a/124a/126a are made of the same material type. In some embodiments, each band 122a, 124a and 125a is made of a different material. In some embodiments, when worn, the entire upper band 124a, the middle band 122a and the lower band 126a are substantially in contact with each other and form a continuous layer around the body of the patient. By substituting a single-piece band, with three bands, the patient can adjust each portion of the spine brace system 100, e.g., lower, upper or middle, independently, which results in a better fit and higher stability of the spine brace system 100 to accommodate the contours and circumference of the patient. Further, the three-band configuration described herein enables the patient to tighten each portion (band) of the spine brace system 100 separately, without exerting excessive compressive force on other portions of the body of the patient covered by the spine brace system 100. Additionally, or in the alternative, in some embodiments, portions of the upper band 124a and the lower band 126a may overlap the middle band 122a. Similarly, in some embodiments, portions of the middle band 122a may overlap the upper band 124a and the lower band 126a.

As shown, the middle band 122a, may include an upper fastening mechanism 129a and a lower fastening mechanism 129b. The upper and lower fastening mechanisms 129a and 129b may be used to fasten the corresponding band. As a non-limiting example, the fastening mechanisms 129a, 129b may constitute complementary unbroken loop (UBL) and hook fastener pairs .

Referring to **FIG. 1B****,** a perspective inside view of an exemplary embodiment of a spine brace system 100 of **FIG. 1A** is shown. As shown, the middle band 122a may include a middle fastening mechanism 129c. Each fastening mechanism may be used to fasten the corresponding band. As a non-limiting example, the fastening mechanism 129c is a hook and loop fastener such as VELCRO^{®}. Since each band 122a, 124a and 126a can operate independently, e.g., the length of each band 122a, 124a and 126a is independent of the other bands, the patient can adjust each of the bands 122a, 124a and 126a separately. This can improve the comfort of the patient since they can adjust the length of each section, i.e., band, based on the needs. For example, the patient may need to increase the length of the upper band 124a after a meal, which can be done by only changing a length of the upper band 124a while the lengths of the other two bands 122a and 126a remain unchanged. While **FIG 1A** and **FIG. 1B** illustrate that the lower and upper fastening mechanisms 129a and 129b are disposed on an opposite side of the middle fastening mechanism 129c, the upper, lower and middle fastening mechanisms 129a, 129b and 129c can be disposed on a same side.

In some embodiments, each band 122a, 124a and 126a folds back on itself by passing through a slot. The details of the slots will be described below. Generally, the patient can pull a portion of a band 122a, 124a, 126a that passes through the slot associated with that band to shorten the band. There may be a fastening region (e.g., region with a fastener complementary with fastening mechanisms 129a, 129b or 129c) disposed on each band 122a, 124a, 126a extending from one end of the band to the other end of the band.

As a non-limiting example, in order to shorten the length of a band (e.g., upper band 126a), the patient can pull the folded portion of the band 126a and couple/attach the pulled folded portion to a point closer to the anterior 110 of the arm 105 or 106 forming a portion of the spine brace system 100 on the fastening region 129a of the band 126a. Similarly, in order to loosen the band (e.g., upper band 126a), the patient can move the folded portion of the band 126a and couple/attach the folded portion to a point closer to the posterior of the arm 105 or 106 forming a portion of the spine brace system 100. Because the bands 122a, 124a and 126a can move independently, the location where a band is folded back on itself and fastened, e.g., fastening mechanism 129a, can be different from the location where the other bands are folded back and fastened, e.g., fastening mechanisms 129b and 129c. Additionally, or in the alternative to the embodiment described above, a hook and loop fastening mechanism may be used for fastening the folded portion of the band 122a, 124a or 126a to the unfolded portion of that band.

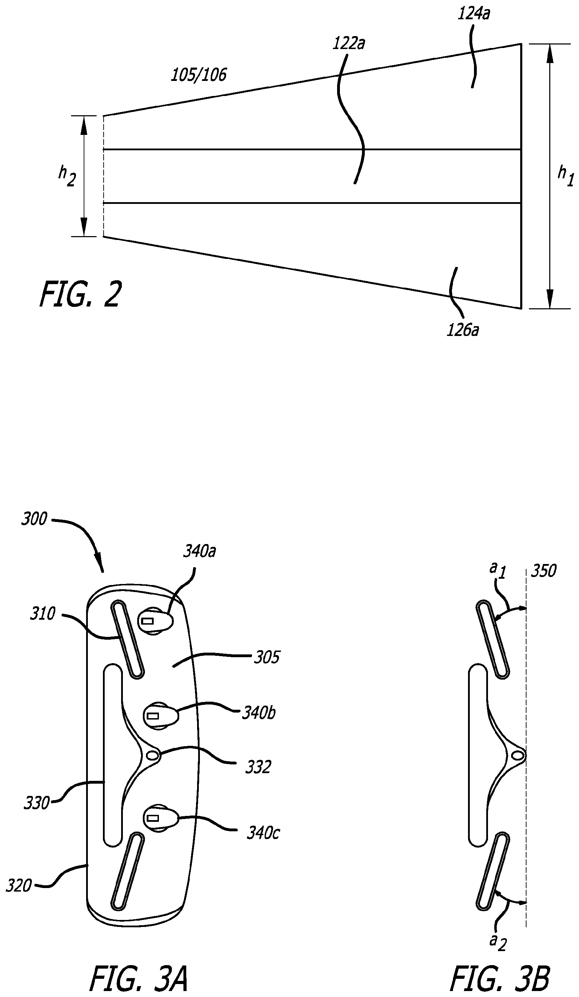

Referring to **FIG. 2****,** a perspective side view of an exemplary embodiment of a spine brace system 100 of **FIG. 1A** and **FIG. 1B** is shown. As shown, while the middle band 122a has a substantially constant width, the overall spine brace system 100 is slightly tapered towards its end. As shown, each spine brace arm 105 and 106 is slightly tapered laterally from the one side (e.g., the posterior of the spine brace system 100 as created by tapering of the arm 105/106 of the spine brace system 100) to the other side (e.g., the anterior of the spine brace system 100 as created by tapering of the arm 105/106 of the spine brace system 100).

According to one embodiment of the disclosure, the slightly tapered laterally-extending spine brace system 100 may form as a result of the angled configuration of the upper slot 310 and the lower slot 320 with respect to the middle slot 330 formed as an adjustment mechanism 300 illustrated in **FIGS. 3A-3B****.** In such embodiments, the upper band 124a is extended slightly downward to couple (e.g., "touch") the middle band 122a from the posterior of the brace system 100 towards the anterior of the spine brace system 100 (and anterior portion of arm 105 or 106). This coupling allows the upper band 124a to come into physical contact with the middle band 122a while still retaining their co-planar orientation. Similarly, the lower band 126a is extended slightly upward to couple (e.g., "touch") the middle band 122a from the posterior of the spine brace system 100 to the anterior of the spine brace system 100. As a result, the portion of the spine brace system 100 extending closer to the posterior portion of each arm 105 or 106 has a height h₁ which is larger than the height h₂ of the portion of the spine brace system 100 extending closer to the anterior portion 110 of the arm 105 or 106.

Herein, as shown in **FIG. 2****,** the band configuration improves the efficiency and comfort of the patient since while the posterior portion and two sides of body of the patient are supported by larger cross section of the spine brace system 100, the abdominal area of the patient, which does not need the support of the spine brace system 100, is covered with a smaller cross section of the spine brace system 100. Moreover, a smaller cross section transforms the unidirectional force exerted by the conventional spine brace to a triangulated force which can increase the stability of each arm 105 and 106, and thus the spine brace system 100 as a whole.

As shown in **FIG. 3A****,** a top view of an exemplary embodiment of an adjustment mechanism 300 featuring a pulley base 305 and slots implemented with the adjustable multi-band spine brace system 100 of **FIG. 1A** and **FIG. 1B** is shown. The adjustment mechanism 300 may allow for independent adjustment of the upper band 124a, the middle band 122a, and the lower band 126a is shown. The adjustment mechanism 300 may include a pulley base 305, and a plurality of slots 310, 330, 320, for the upper band 124a, the middle band 122a and lower band 126a of **FIG. 1A** or **FIG. 1B****,** respectively. In various embodiments, while the pulley base 305 may be used for tightening the spine brace, the plurality of slots 310, 320 and 330 may be used to adjust the bands of the spine brace system 100. The pulley base 305 may include a plurality of pulleys 340a, 340b, 340c disposed and a cord for causing tension to pull complementary pulley base pairs for the arms 105/106 of the spine brace system 100 (not shown).

Referring still to **FIG. 3****,** the (middle) slot 330 for the middle band 122a of **FIGS. 1A-****1B** and **FIG. 2** is disposed between the (upper) slot 310 for the upper band 124a of **FIGS. 1A-****1B** and **FIG. 2** and the (lower) slot 320 for the lower band 126a of **FIGS. 1A-1B** and **FIG. 2****.** As shown in **FIG. 3B****,** the upper slot 310 and lower slot 320 may be angled with respect to the middle slot 330, namely angle a₁ and angle a₂. The angled configuration a₁ of the upper slot 310, rotated in a counter-clockwise direction from vertical as shown by a dashed line 350, allows the upper band 122a to extend slightly downward to couple to the middle band 122a from the posterior of the spine brace system 100 to the anterior of the spine brace system 100. Similarly, the angled configuration a₂ of the lower slot 320, rotated in a clockwise direction from vertical, allows the lower band 122a to extend slightly upward to couple to the middle band 122a from the posterior of the spine brace system 100 to the anterior of the spine brace system 100. In some embodiments, the clockwise angle of rotation of the lower slot 320 is the same as the counter-clockwise angle of rotation of the upper slot 310. That is, the absolute angular value of a1 is the same as the absolute angular value of a2.

The rotation angle of the upper slot 310 and the rotation angle of the lower slot 320 with respect to the middle slot 330 are so determined that, upon folding each band, a folded portion of the upper band 124a would be fastened to the upper band 124a. Additionally, or in the alternative, a folded portion of the middle band 122a would be fastened to the middle band 122a. Similarly, a folded portion of the lower band 126a would be fastened to the lower band 126a. This ensures that the bands 122a, 124a and 126a, in their folded position, maintain the continuous configuration, in that, the folded upper and folded lower bands come into physical contact with the middle band while still retaining their co-planar orientation.

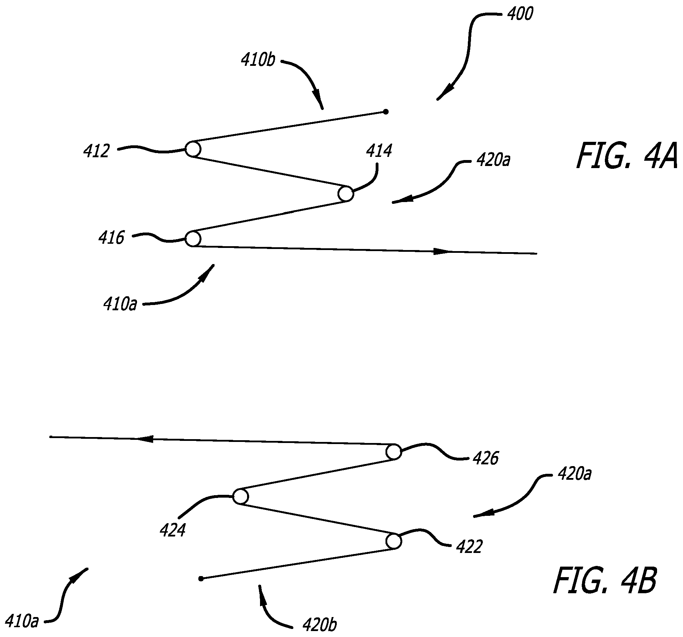

**FIG. 4A** is a top view of an exemplary embodiment of the operability of a first pulley base 410a of a pulley system 400 implemented with the adjustable multi-band spine brace system 100 of **FIG. 1A** and **FIG. 1B****.** Similarly, **FIG. 4B** is a top view of an exemplary embodiment of the operability of a second pulley base 420a of the pulley system 400 implemented with the adjustable multi-band spine brace system 110 of **FIG. 1A** and **FIG. 1B****.** In some embodiments, the adjustment mechanism features a tightening mechanism. The tightening mechanism may include the pulley system 400 to tighten each of the cords on the first arm 105 or second arm 106 of the spine brace system 100, independently.

Herein, the first pulley base 410a of the pulley system 400, represented as pulley base 305 of **FIG. 3****,** may feature a plurality of pulleys (e.g., pulleys 412 and 416) interconnected with a pulley 414 of the second pulley base 420a via a cord (e.g., 410b) as shown in **FIG. 4A****.** The second pulley base 420a, representing a 180° degree x-y axis rotation of the first pulley base 410a features a plurality of pulleys (e.g., 422 and 426) interconnected with a pulley 424 of the first pulley base 410a via the cord (e.g., 420b) as shown in **FIG. 4B****.** The patient can tighten a cord 410b or 420b by pulling the cord 410b or 420b. In some embodiments, the tightening mechanism features one or more of the pulley system 400 in combination with other fastening features such as various the loop and hook fasteners operating in concert. In some embodiments, while the pulleys 412-416 and 422-426 are used to tighten the spine brace system 100, the slots 310, 330 and 320 of **FIGS. 3A-3B** are used to adjust the length of each band 124a, 122a and 126a of the spine brace system 100 independently.

The pulley system may rely on three pulleys and the cord. That is, the pulleys, an anchor on the pulley base, and the cord form a 4:1 pulley system. In such 4:1 pulley system configurations, a first end of the cord is coupled directly to the middle band and enters a first end of the adjustment mechanism 300. After passing through designated pulleys, the cord 410b or 420b extends outwardly from a second end of the adjustment mechanism. In such embodiments, tightening of the spine brace system 100 is performed by pulling the second end of the cord 410b or 420b.

In some embodiments, two tightening mechanisms, each featuring a pulley system, are disclosed. A first tightening mechanism allows for independent tightening of the middle band of the first arm of the spine brace system. With a two-tightening mechanism configuration, once the patient tightens the middle band of a particular arm of the spine brace, the corresponding portion of the tightening mechanism of the two tightening mechanism becomes closer to the other tightening mechanism. Alternatively, when the patient tightens both tightening mechanisms, the entire two tightening mechanisms become closer together.

Referring back to **FIG. 3A****,** in some embodiments, a lower part of the upper slot 310 may be arranged to slightly overlap with an upper part of the middle slot 330 so that the upper slot 310 is interposed between the middle slot 330 and a first pulley 340a mounted on the pulley base 305. In some embodiments, an upper part of the lower slot 320 may be arranged to slightly overlap with a lower part of the middle slot 330 so that the upper part of the lower slot 320 is interposed between the middle slot 330 and a third pulley 340c mounted on the pulley base 305. The middle slot 330 may have an opening 332, referred to as a T-shaped opening. The T-shaped opening 332 facilitates adjustment of the middle band 122a of **FIGS. 1A-1B** and **FIG. 2****.** The T-shaped opening 332 narrows slightly in a center region of the tightening mechanism which can further guide a cord.

In some embodiments, the spine brace system 100 may include a middle cord 122b for adjusting tension of the middle band 122a. In such embodiments, the spine brace system further includes the adjustment mechanism that has slots for the middle band 122a. In some embodiments, the tightening mechanism includes slots for each of the upper, middle and lower bands. Optionally, the upper and lower slots may be angled with respect to the middle slot.

Referring back to **FIG. 4A** and **FIG. 4B****,** as shown, the first pulley base 410a is associated with a tightening mechanism which is configured to tighten the first arm 105 of the spine brace system 100 (see **FIG. 1A****).** Similarly, the second pulley base 420a is associated with the tightening mechanism, which is configured to tighten the second arm 106 of the spine brace system 100 (see **FIG. 1B****).** Each pulley base 410a or 420b relies on a cord and a plurality of pulleys. For example, the first pulley base 410a includes the first cord 410b and the first plurality of pulleys 412, 414 and 416. By tightening the first cord 410b that passes through each of the pulleys 412, 414 and 416, the patient can tighten the first arm 105 of the spine brace system 100. Similarly, the second pulley base 420a includes the second cord 420b and the second plurality of pulleys 422, 424 and 426. By tightening the second cord 420b that passes through each of the pulleys 422, 424 and 426, the patient can tighten the second arm 106 of the spine brace system 100.

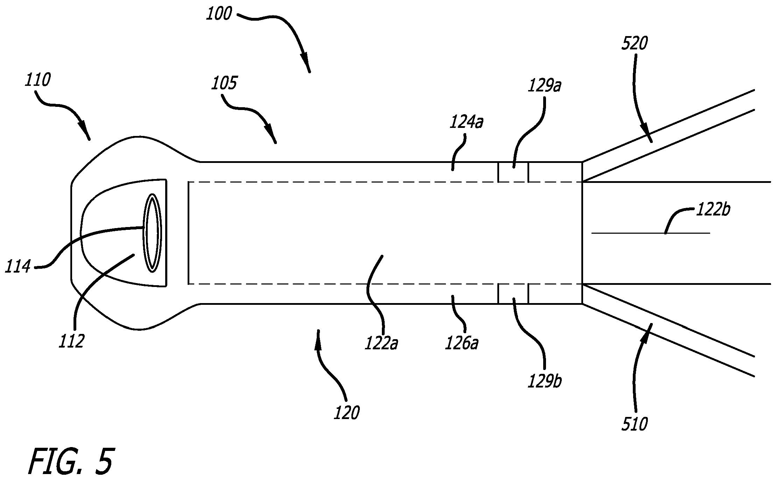

**FIG. 5** is a perspective outside view of an exemplary embodiment of an adjustable multi-band spine brace system. In some embodiments, when worn, the band 122a and 124a are so arranged that one edge of the upper band 124a is in contact with one edge of h middle band 122a while the upper band 124a and the middle band 122a do not overlap. Similarly, the band 122a and 126a may be so arranged that one edge of the lower band 126a is in contact with one edge of h middle band 122a while the lower band 126a and the middle band 122a do not overlap. Additionally, or in the alternative, in some embodiments, when worn, the band 122a and 124a are so arranged that the upper band 124a and the middle band 122a overlap in the lateral portion 120. Similarly, the band 122a and 126a may be so arranged that the lower band 126a and the middle band 122a overlap in the lateral portion 120.

In some embodiments, the upper band 124a and the middle band 122a are so arranged that portions of the upper band 124a extending towards the posterior portion of the spine brace 100 are separate from portions of the middle band 122a extending towards the posterior portion of the spine brace 100, as shown by the region 520 in **FIG. 5****.** Additionally, or in the alternative, in some embodiments, the lower band 126a and the middle band 122a are so arranged that portions of the lower band 126a extending towards the posterior portion of the spine brace 100 are separate from portions of the middle band 122a extending towards the posterior portion of the spine brace 100, as shown by the region 510 in **FIG. 5****.**

### III. OPTIONAL ARCHITECTURE - INTERNAL LATERAL PANEL WITHIN BRACE ARM

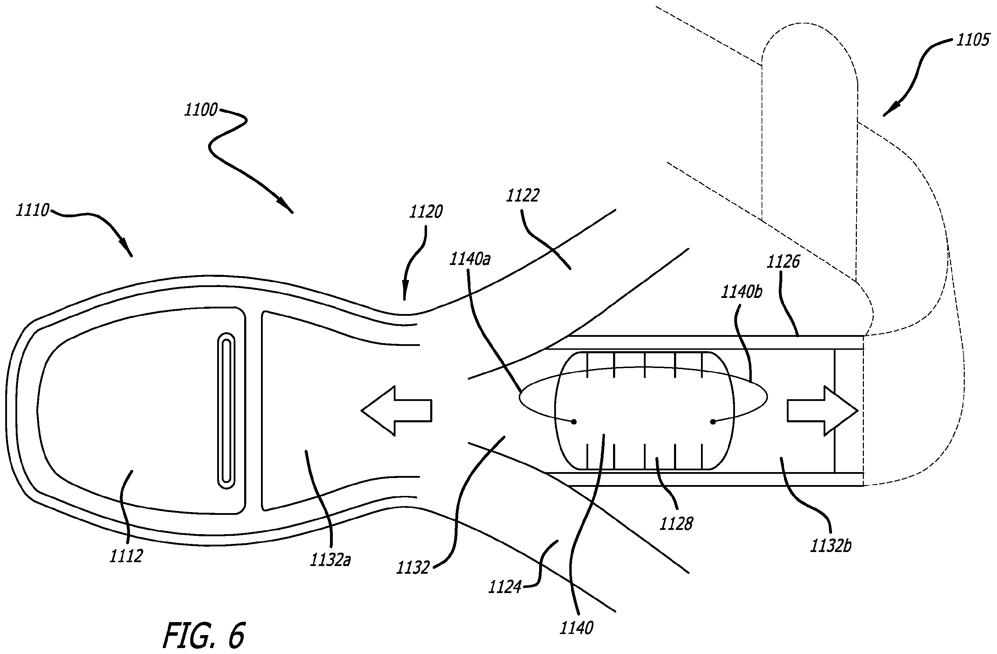

Referring to **FIG. 6****,** a perspective outside view of a first exemplary embodiment of an arm 1100 of a brace with one or more slidable, internal lateral panels is shown. It should be noted that, the arm 1100 with a slidable, internal lateral panel is a portion of a brace 1105 (e.g., LSO, TLSO, CTLSO, etc.) that includes two arms, each covering one side of the wearer. Each arm (e.g., arm 1100 of the brace 1105) with slidable, internal lateral panel includes an anterior portion 1110 (i.e., a front portion), a lateral portion 1200, and a posterior portion (i.e., a back portion, not shown). The posterior portion is oriented, when worn, to generally reside or is substantially in parallel with a back plane of the patient. The posterior portion may provide support along a thoracic and lumbar regions of the patient's spine. The lateral portion 1200 may cover the sides of the patient. Similarly, the anterior portion 1110 is oriented, when worn, to generally reside or is substantially in parallel with a frontal plane of the patient. The anterior portion 1110 may be in contact with the abdominal region of the patient's spine.

Each arm (e.g., arm 1100) may include a coupling section 1112, as shown in **FIG. 6****.** Each coupling section 1112 is configured to removably attach/couple to the other coupling section. Regardless of the size and shape, the coupling section of the arms are so formed that complement each other and, when attached/coupled together, form a uniformly closed brace which circumferentially surrounds the body of the patient. Therefore, in some embodiments, the anterior portion 1110 of the two arms of the brace (not shown) may be removably coupled together by using a hook and loop coupling. As a non-limiting example, the coupling section 1112 may include a VELCRO^{®} strip or another type of hook fastener while the coupling section for the other arm could include a loop fastener. In some embodiments, the anterior portion 1110 may further include a slot. In such embodiments, an additional band may be provided in one arm which can be inserted into the slot of the other arm to couple the anterior portions of the arms.

As shown, the lumbar region of a wearer features a first band 1126, an upper band 1122, and a lower band 1124. The first band 1126 is disposed between the upper band 1122 and the lower band 1124. The arm 1100 with slidable, internal lateral panel may feature a panel 1128 which is disposed at a lateral side of the wearer and coupled to the first band 1126. The panel 1128 may be slidably disposed at a lateral side of the wearer and coupled to the first band 1126. The first band 1126 has a horizontally extending channel 1132, within which the panel 1128 moves. The brace with slidable, internal lateral panel 1000 may feature a loop connector 1140 coupled to the panel 1128. Two ends of the loop connector 1140 may be connected, i.e., coupled, to the panel 1128, which may assist in imposing restrictions on lateral movement of the panel 1128. The first end 1140a of the loop connector 1140 may be connected to the panel 1128 on a first end of the panel 1128 which is substantially close to a first end of the channel 1132, and a second end 1140b of the loop connector 1140 may be connected to the panel 1128 which is substantially close to a second end of the channel 1132.

In various embodiments of the present disclosure, the panel 1128 is configured to provide lateral support for the wearer. Thus, the panel 1128 features a rigid material to provide such support. The loop connector 1140 may be tethered to the panel 1128. Moving the connector loop 1140 causes moving the panel 1128 laterally, which adjusts a location of the panel 1128 around the lumbar region of the wearer, which in turn provides lateral support for the medial region, i.e., sides, of the wearer. As a non-limiting example, the location of the panel may be adjusted based on the size of the wearer of the brace. In some embodiments, while a first position of the panel is desirable, e.g., comfortable, for a first wearer, that position is undesirable, e.g., uncomfortable, for another wearer who is larger in size compared to the first wearer, and vice versa.

The panel 1128 may include a rigid material. As a non-limiting example, the panel 1128 may include a polymer with suitable stiffness to be used as the panel 1128. The loop connector 1140 may be tethered to the panel 1128, such as D-ring style or other types of connection apertures formed in the polymer. Movement of the loop connector 1140 causes lateral movement of the panel 1128, which adjusts a location of the slidable, internal lateral panel 1128 around the lumbar region of the wearer. In some embodiments, the loop connector 1140 and the panel 1128 may form a closed loop.

The first band 1126 of the brace with slidable, internal lateral panel 1000 may have a channel 1132. The channel 1132 is horizontally extending from the first side of the first band 1126 (closer to the first side of the channel, 1132a) to the second side of the first band 1126 (closer to the second side of the channel 1132b). The panel 1128 is configured to move, i.e., slide, within the first band 1126 and along the channel 1132. Thus, the length of the channel 1132 may determine the overall change in the location of the panel within the slidable, internal lateral panel 1000. In some embodiments, the channel 1132 may be used for cord/tube management. As a non-limiting example, one or more tubes, cords, cables or wires may be inserted into the channel 1132. For example, one or more wires, cables, cords or other interconnects connected to medical equipment to monitor patient's vitals and/or tubes, e.g., catheters, can be inserted into the channel for wire/tube management.

The wearer of the brace may pull the loop connector 1140 from the first end 1132a to the second end 1132b of the channel 1132 to change, i.e., adjust, the location of the panel 1128. In other words, moving, i.e., sliding, the panel 1128 by pulling the loop connector 1140 from the first end 1132a to the second end 1132b causes adjustment of the location of the panel 1128 around the lumbar region of the wearer.

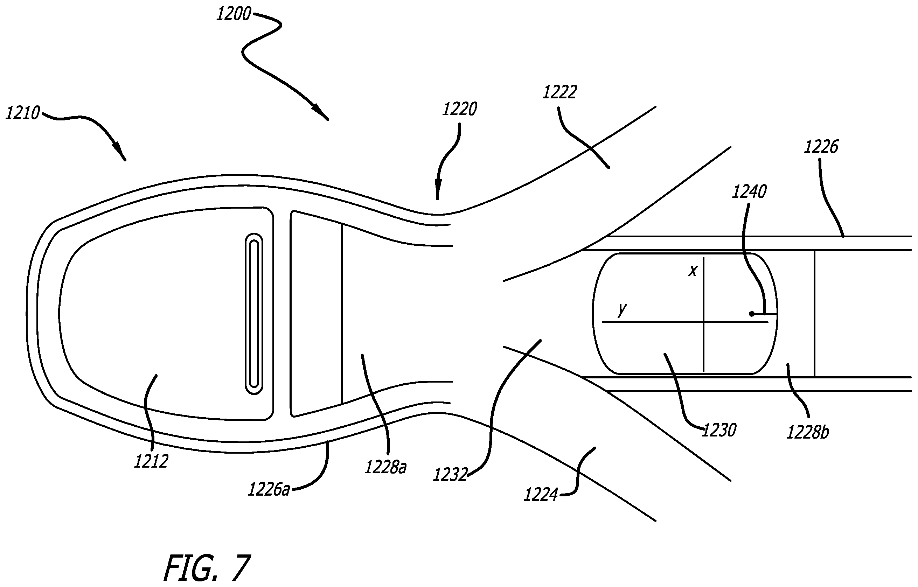

Referring to **FIG. 7****,** a perspective side view of a second exemplary embodiment of an arm 1200 with one or more slidable, internal lateral panels of **FIG. 6** is shown. As shown, the arm 1200 with a slidable, internal lateral panel may feature a first band 1226, and a panel 1230 slidably disposed within a channel 1232 on the first band 1226. A lateral movement of the panel 1230 in the channel 1232 may be constrained by a first boundary 1228a disposed on a first end of the channel 1232, and a second boundary 1228b disposed on a second end of the channel 1232. In some embodiments, the first and second boundaries 1228a and 1228b are formed by sewing the first band 1226. In some embodiments, the first and second boundaries 1228a and 1228 are disposed within the channel 1232. The size of the panel 1230 is so selected that the panel 1230 can only laterally slide within the channel 1232. To that end, regardless of the lateral dimension of the panel 1230, i.e., "y" direction, the vertical dimension of the panel 1230, i.e., "x" direction, is so selected that the panel's movement is constrained in only one direction, i.e., lateral y-direction.

In some embodiments, the arm 1200 with slidable, internal lateral panel may feature a handle member 1240 disposed on the panel 1230. The handle member 1240 may be a tube knitted to the panel, a leash or any other suitable handle. The arm 1200, being a portion of the lumbar brace (not shown), may further feature an upper band 1222 and a lower band 1224, which the first band 1226 may be disposed between the upper band 1222 and the lower band 1224. In such embodiments, the lateral movement of the panel 1230 between the first boundary 1228a and the second boundary 1228b adjusts the location of the panel 1230 around the lumbar region of the wearer.

In some embodiments, moving the handle member 1240 may cause the panel 1230 to move horizontally within the channel 1232. The handle member 1240 may be a tube knitted to the panel 1230. Additionally, or in the alternative, the handle member 1240 may be a leash attached to the panel 1230. It should be noted that, the handle member 1240 may be any kind of suitable attachment.

Referring back to **FIG. 6** now, the arm 1100 with slidable, internal lateral panel 1128 further features the first band 1126 disposed between an upper band 1124 and a lower band 1122. The first band 1126, the upper band 1124 and the lower band 1122 are attached/coupled in one end (e.g., at the posterior portion of the brace with slidable, internal lateral panel 1000). The first band 1126, the upper band 1124 and the lower band 1122 may operate independent of each other. Each of the upper band 1124, the lower band 1122 and the first band 1126 are made of flexible materials that can flex and/or bend around the circumference body of the patient. When worn, at least portions of the upper band 1124, the first babe 1126 and the lower band 1124 may be in contact with each other. In various embodiments, each band is made of different materials. For example, the upper and lower bands are made of a same material which is substantially softer than the material which the middle band is made of. Alternatively, at least two of the bands are made of the same materials. In some embodiments, each band is made of a different material.

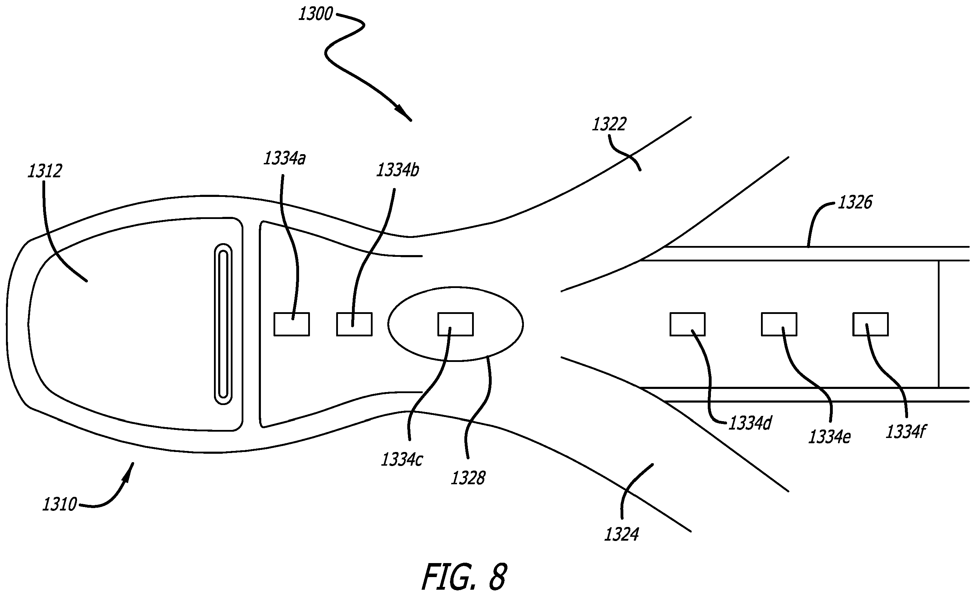

Referring to **FIG. 8****,** a perspective outside view of a third exemplary embodiment of an arm 1300, being a portion of the brace, with one or more slidable, internal lateral panels of **FIG. 6** is shown. As shown, the arm 1300 with a slidable, internal lateral panel may feature a plurality of openings 1334a, 1334b, 1334c, 1334d, 1334e, 1334f disposed on the first band 1326. In such embodiments, the panel 1328 may laterally slide between the plurality of openings 1334a, 1334b, 1334c, 1334d, 1334e, 1334f. The first band 1326 may include the horizontally extending channel, which may extend along the entire length of the first band.

The plurality of openings 1334a, 1334b, 1334c, 1334d, 1334e, 1334f are disposed along the channel (which is similar to the channel 132 in **FIG. 6****,** but is not shown for the sake of simplicity) and the panel 1328 slides within the channel and between the plurality of openings 1334a, 1334b, 1334c, 1334d, 1334e, 1334f. The first band 1326 is disposed between the upper band 1322 and the lower band 1324. According to some embodiments, sliding the panel 1328 between the plurality of openings 1334a, 1334b, 1334c, 1334d, 1334e, 1334f adjusts the location of the panel 1328 around the lumbar region of the wearer. For example, the wearer, i.e., the patient, can adjust the location of the panel to properly fit the brace by moving the panel towards any of the openings 1334a, 1334b, 1334c, 1334d, 1334e and 1334f.

As an optional feature, the panel 1328 may include one or more areas along a top surface of the panel 1328 that include tactile material. This tactile material would allow for better gripping of a finger of the wearer to adjust the lateral positioning of the panel 1328 around the lumbar region.

In the foregoing description, the invention is described with reference to specific exemplary embodiments thereof. For example, the telescopic lateral panels and adjustable belt member combination, operating with a pulley subsystem, may be deployed within a LSO orthopedic brace with an architecture different than the orthopedic brace described above. Hence, it will be evident that certain components may be deployed within different types of orthopedic braces and various modifications and changes may be made thereto without departing from the scope of the invention as set forth in the appended claims.

## Claims

1. A spine brace comprising:
a plurality of arms including a_first arm (105) and a second arm (106), wherein each of the plurality of arms comprises:
an upper band (124a);
a lower band (126a), and
a middle band (122a) disposed between the upper band (124a) and the lower band (126a); and
an adjustment mechanism (300) that allows for independent adjustment of the upper band, the middle band and the lower band, **characterized in that** the adjustment mechanism (300) comprises a pulley base (305) including a plurality of pulleys (340a, 340b, 340c), wherein the pulley base (305) includes a first slot (310) for receiving the upper band, a second slot (320) for receiving the lower band, and a third slot (330) for receiving the middle band,
wherein the first slot (310) is rotated in a counter-clockwise direction from vertical to allow the upper band to extend downward towards the middle band and the second slot (320) is rotated in the counter-clockwise direction from vertical to allow the lower band to extend upward towards the middle band.

2. The spine brace of claim 1, wherein the middle band (122a) includes a channel (1132) and a panel (1128) disposed within the channel, a lateral movement of the panel in the channel is constrained by a first boundary at a first end of the channel and a second boundary at a second end of the channel.

3. The spine brace of claim 1, wherein the third slot (330) has a T-shaped opening.

4. The spine brace of claim 3, wherein the opening narrows in a center of the adjustment mechanism (300).

5. The spine brace of claim 1, wherein the first arm (105) of the plurality of arms further comprises:
a cord coupled to the first arm (105), wherein the cord is configured to pass around each of the plurality of pulleys and to independently tighten each of the upper band, the middle band, and the lower band associated with the first arm.

6. The spine brace of claim 5, wherein the plurality of pulleys comprises at least three pulleys and an anchor for the cord.

7. The spine brace of claim 1, wherein the third slot (330) for the middle band includes an opening that narrows at a center region of the opening and is longer in length than an opening of the first slot (310) associated with the upper band and the second slot (320) associated with the lower band.

8. The spine brace of claim 1, wherein each of the upper band, the lower band and the middle band are adapted to be independently tightened by changing a size of a looped end securely attached to the first slot, the second slot and the third slot of the attachment mechanism.

9. The spine brace of claim 1, wherein the middle band (122a), the upper band (124a) and the lower band (126a) collectively form a first arm of the plurality of arms.

10. The spine brace of claim 2, wherein a loop connector is coupled to the panel to facilitate movement of the panel within the channel.

11. The spine brace of claim 2, wherein the middle band (122a) further includes a plurality of openings positioned along a lateral side surface on the middle band to allow for movement of the panel by tactile contact with the panel and adjustment of the panel.

12. The spine brace of claim 1, wherein the first slot (310) and the second slot (320) are angled with respect to the third slot (330) such that,
a folded portion of the upper band is configured to be fastened to the upper band,
a folded portion of the middle band is configured to be fastened to the middle band, and
a folded portion of the lower band is configured to be fastened to the lower band.

## Patentansprüche

1. Rückenstütze, umfassend:
eine Vielzahl von Armen, die einen ersten Arm (105) und einen zweiten Arm (106) einschließen, wobei jeder der Vielzahl von Armen Folgendes umfasst:
ein oberes Band (124a);
ein unteres Band (126a), und
ein mittleres Band (122a), das zwischen dem oberen Band (124a) und dem unteren Band (126a) angeordnet ist; und
einen Einstellmechanismus (300), der unabhängige Einstellung des oberen Bands, des mittleren Bands und des unteren Bands ermöglicht, **dadurch gekennzeichnet, dass**
der Einstellmechanismus (300) eine Rollenbasis (305) umfasst, die eine Vielzahl von Rollen (340a, 340b, 340c) einschließt, wobei die Rollenbasis (305) einen ersten Schlitz (310) zum Aufnehmen des oberen Bands, einen zweiten Schlitz (320) zum Aufnehmen des unteren Bands und einen dritten Schlitz (330) zum Aufnehmen des mittleren Bands einschließt,
wobei der erste Schlitz (310) in einer Richtung gegen den Uhrzeigersinn von der Vertikalen gedreht ist, um zu ermöglichen, dass sich das obere Band nach unten zum mittleren Band hin erstreckt, und der zweite Schlitz (320) in einer Richtung gegen den Uhrzeigersinn von der Vertikalen gedreht ist, um zu ermöglichen, dass sich das untere Band nach oben zu dem mittleren Band hin erstreckt.

2. Rückenstütze nach Anspruch 1, wobei das mittlere Band (122a) einen Kanal (1132) und eine Platte (1128), die innerhalb des Kanals a angeordnet ist, einschließt, wobei eine seitliche Bewegung der Platte in dem Kanal durch eine erste Begrenzung an einem ersten Ende des Kanals und eine zweite Begrenzung an einem zweiten Ende des Kanals eingeschränkt ist.

3. Rückenstütze nach Anspruch 1, wobei der dritte Schlitz (330) eine T-förmige Öffnung aufweist.

4. Rückenstütze nach Anspruch 3, wobei sich die Öffnung in einer Mitte des Einstellmechanismus (300) verengt.

5. Rückenstütze nach Anspruch 1, wobei der erste Arm (105) der Vielzahl von Armen weiter Folgendes umfasst:
eine Schnur, die mit dem ersten Arm (105) gekoppelt ist, wobei die Schnur so ausgebildet ist, dass sie um jede der Vielzahl von Rollen herumgeht und unabhängig voneinander jedes des oberen Bands, des mittleren Bands und des unteren Bands, die dem ersten Arm zugeordnet sind, festzieht.

6. Rückenstütze nach Anspruch 5, wobei die Vielzahl von Rollen mindestens drei Rollen und einen Anker für die Schnur umfasst.

7. Rückenstütze nach Anspruch 1, wobei der dritte Schlitz (330) für das mittlere Band eine Öffnung einschließt, die sich in einem Mittelbereich der Öffnung verengt und in der Länge länger ist als eine Öffnung des ersten Schlitzes (310), der dem oberen Band zugeordnet ist, und des zweiten Schlitzes (320), der dem unteren Band zugeordnet ist.

8. Rückenstütze nach Anspruch 1, wobei jedes des oberen Bands, des unteren Bands und des mittleren Bands so eingestellt wird, dass sie unabhängig voneinander durch Ändern einer Größe eines geschlauften Endes, das sicher an dem ersten Schlitz, dem zweiten Schlitz und dem dritten Schlitz des Befestigungsmechanismus befestigt ist, festgezogen werden.

9. Rückenstütze nach Anspruch 1, wobei das mittlere Band (122a), das obere Band (124a) und das untere Band (126a) gemeinsam einen ersten Arm der Vielzahl von Armen bilden.

10. Rückenstütze nach Anspruch 2, wobei ein Schlaufenverbinder mit der Platte gekoppelt ist, um Bewegung der Platte innerhalb des Kanals zu erleichtern.

11. Rückenstütze nach Anspruch 2, wobei das mittlere Band (122a)weiter eine Vielzahl von Öffnungen einschließt, die entlang einer seitlichen Seitenoberfläche auf dem mittleren Band positioniert sind, um Bewegung der Platte durch taktilen Kontakt mit der Platte und Einstellung der Platte zu ermöglichen.

12. Rückenstütze nach Anspruch 1, wobei der erste Schlitz (310) und der zweite Schlitz (320) in Bezug auf den dritten Schlitz (330) so angewinkelt sind, dass,
ein gefalteter Abschnitt des oberen Bands so ausgebildet ist, dass er an dem oberen Band befestigt wird,
ein gefalteter Abschnitt des mittleren Bands so ausgebildet ist, dass er an dem mittleren Band befestigt wird, und
ein gefalteter Abschnitt des unteren Bands so ausgebildet ist, dass er an dem unteren Band befestigt wird.

## Revendications

1. Orthèse dorsale comprenant :
une pluralité de bras, incluant un premier bras (105) et un deuxième bras (106), dans laquelle chaque bras de la pluralité de bras comprend :
une bande supérieure (124a) ;
une bande inférieure (126a), et
une bande du milieu (122a), agencée entre la bande supérieure (124a) et la bande inférieure (126a) ; et
un mécanisme de réglage (300), qui permet un réglage indépendant de la bande supérieure, de la bande du milieu et de la bande inférieure, **caractérisée en ce que**
le mécanisme de réglage (300) comprend une base pour poulies (305), incluant une pluralité de poulies (340a, 340b, 340c), dans laquelle la base pour poulies (305) inclut une première fente (310) pour recevoir la bande supérieure, une deuxième fente (320) pour recevoir la bande inférieure, et une troisième fente (330) pour recevoir la bande du milieu,
dans laquelle la première fente (310) est tournée dans le sens antihoraire par rapport à la verticale pour permettre à la bande supérieure de s'étendre vers le bas en direction de la bande du milieu, et la deuxième fente (320) est tournée dans le sens antihoraire par rapport à la verticale pour permettre à la bande inférieure de s'étendre vers le haut en direction de la bande du milieu.

2. Orthèse dorsale selon la revendication 1, dans laquelle la bande du milieu (122a) inclut un canal (1132) et un panneau (1128) agencé à l'intérieur du canal, le mouvement latéral du panneau dans le canal étant limité par une première limite à une première extrémité du canal et une deuxième limite à une deuxième extrémité du canal.

3. Orthèse dorsale selon la revendication 1, dans laquelle la troisième fente (330) présente une ouverture en forme de T.

4. Orthèse dorsale selon la revendication 3, dans laquelle l'ouverture se rétrécit au centre du mécanisme de réglage (300).

5. Orthèse dorsale selon la revendication 1, dans laquelle le premier bras (105) de la pluralité de bras comprend en outre :
un cordon relié au premier bras (105), dans laquelle le cordon est configuré pour passer autour de chaque poulie de la pluralité de poulies et pour serrer indépendamment chacune parmi la bande supérieure, la bande du milieu et la bande inférieure associées au premier bras.

6. Orthèse dorsale selon la revendication 5, dans laquelle la pluralité de poulies comprend au moins trois poulies et un ancrage pour le cordon.

7. Orthèse dorsale selon la revendication 1, dans laquelle la troisième fente (330) pour la bande du milieu inclut une ouverture qui se rétrécit au niveau d'une zone centrale de l'ouverture et qui est plus longue que l'ouverture de la première fente (310) associée à la bande supérieure et de la deuxième fente (320) associée à la bande inférieure.

8. Orthèse dorsale selon la revendication 1, dans laquelle chacune parmi la bande supérieure, la bande inférieure et la bande du milieu est adaptée pour être serrée indépendamment en modifiant la taille d'une extrémité formant boucle solidement fixée à la première fente, à la deuxième fente et à la troisième fente du mécanisme de fixation.

9. Orthèse dorsale selon la revendication 1, dans laquelle la bande du milieu (122a), la bande supérieure (124a) et la bande inférieure (126a) forment collectivement un premier bras de la pluralité de bras.

10. Orthèse dorsale selon la revendication 2, dans laquelle un connecteur formant boucle est relié au panneau pour faciliter le mouvement du panneau à l'intérieur du canal.

11. Orthèse dorsale selon la revendication 2, dans laquelle la bande du milieu (122a) inclut en outre une pluralité d'ouvertures positionnées le long d'une surface latérale sur la bande du milieu pour permettre le mouvement du panneau par contact tactile avec le panneau et l'ajustement du panneau.

12. Orthèse dorsale selon la revendication 1, dans laquelle la première fente (310) et la deuxième fente (320) sont inclinées par rapport à la troisième fente (330) de telle sorte que
une partie pliée de la bande supérieure soit configurée pour être fixée à la bande supérieure,
une partie pliée de la bande du milieu soit configurée pour être fixée à la bande du milieu, et
une partie pliée de la bande inférieure soit configurée pour être fixée à la bande inférieure.
